(19) Europäisches Patentamt
European Patent Office
Office européen des brevets

(11) Veröffentlichungsnummer : **0 040 355**
**B1**

(12) # EUROPÄISCHE PATENTSCHRIFT

(45) Veröffentlichungstag der Patentschrift : **25.07.84**

(51) Int. Cl.³ : **C 07 J 13/00**, C 07 J 5/00

(21) Anmeldenummer : **81103421.4**

(22) Anmeldetag : **06.05.81**

(54) **Verfahren zur partiellen Reduktion von C21-Steroidcarbonsäuren und ihren Estern zu C21-Steroidalkoholen.**

(30) Priorität : **16.05.80 AT 2628/80**

(43) Veröffentlichungstag der Anmeldung : **25.11.81 Patentblatt 81/47**

(45) Bekanntmachung des Hinweises auf die Patenterteilung : **25.07.84 Patentblatt 84/30**

(84) Benannte Vertragsstaaten : **BE CH FR LI NL**

(56) Entgegenhaltungen :
**DE-A- 2 558 077**
**DE-B- 1 226 576**
**CHEMICAL PHARMACEUTICAL BULLETIN, Band 24, Nr. 4, April 1976, Seiten 828-831 H. SAKAMOTO et al.: "Chemical modifications of androsta-1,4-diene-3,17-dione. III. The synthesis of (20R)-17alpha,20,21-trihydroxypregna-1,4-dien-3-one and its derivatives"**
**CHEMISCHE BERICHTE, Band 109, Nr. 8, August 1976, Seiten 2954-2955 Weinheim, DE. U. EDER: "Notiz über Testosteron aus 4-Androsten.3,17-dion durch Diisobutylalmuniumhydrid-Reduktion und selektive "in situ"- Oppenauer-Oxidation"**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Preuss, Wolfgang, Dr.**
**Finkenweg 12**
**D-4019 Monheim (DE)**

**EP 0 040 355 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung beschreibt ein verbessertes und insbesondere vereinfachtes Verfahren zur partiellen Reduktion der C 21-Carbonsäuregruppe in entsprechenden Steroidcarbonsäuren mit einer $\Delta^4$-3-on- oder $\Delta^{1,4}$-3-on-Konfiguration im A-Ring des Steroidgerüsts zur entsprechenden C 21-Alkoholfunktion. Gegebenenfalls können auch weitere funktionelle Gruppen im Steroidgerüst vorliegen, die durch diese partielle Reduktion vollständig oder weitgehend unberührt bleiben.

Progesteron (4-Pregnen-3,20-dion) und insbesondere Progesteron-Derivate mit einer Sauerstoff-Funktion in 11-Stellung (11-Oxo, 11α-OH, 11β-OH) der Formeln (1) bis (3)

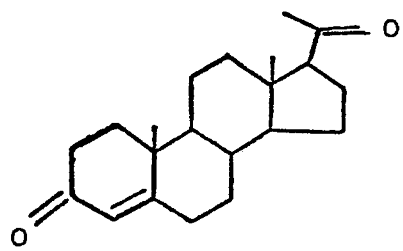

(1)

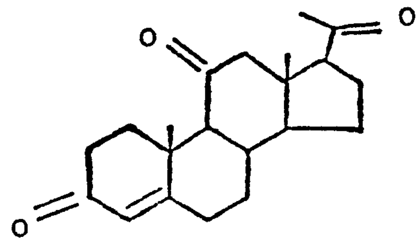

(2)

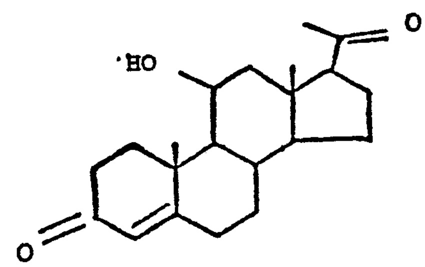

(3)

sind ausgezeichnete und teilweise technisch genutzte Ausgangsmaterialien für die Synthese von Vorstufen des Hydrocortisons (Cortisols) und schließlich des Hydrocortisons selbst. Ein Reaktionsschema zu diesem Weg findet sich z. B. in L.F. Fieser, M. Fieser « Steroide », Seiten 737, 738, Verlag Chemie, Weinheim, 1961. Einzelheiten sind u. a. der dort zitierten Originalliteratur J.A. Hogg et al, J. Am. Chem. Soc. 77, 4436 (1955), sowie den US-PSen 2 683 724 und 2 707 184 zu entnehmen.

Die Reaktionsfolge ist hier am Beispiel des 11-Keto-Progesterons (4) etwas verkürzt wiedergegeben :

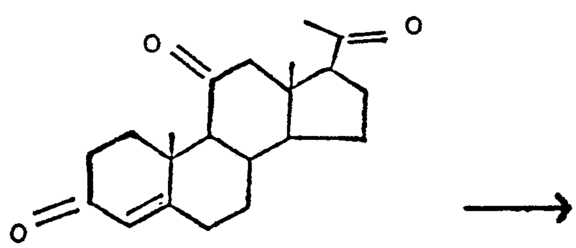

(4)

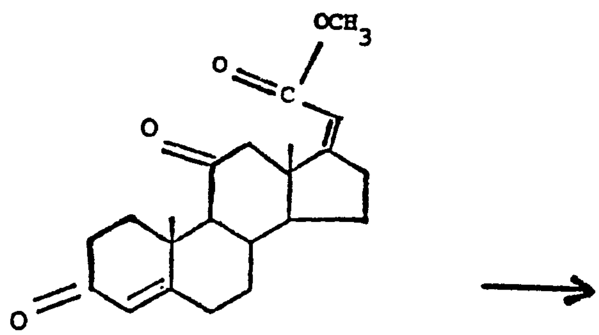

(5)

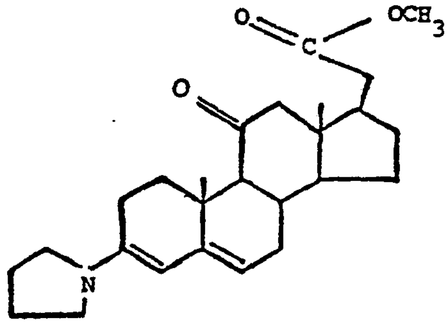

(6)

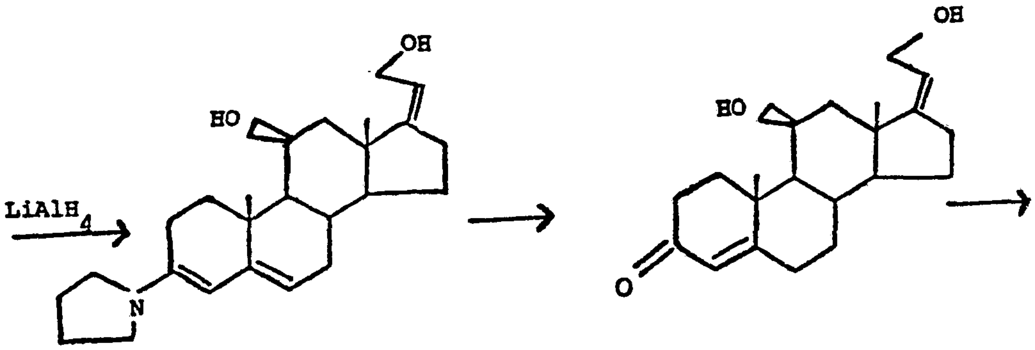


(7) (8)

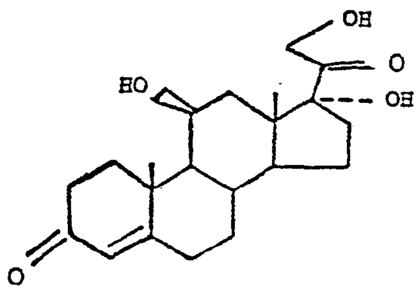

Hydrocortison

Von herausragender Bedeutung für den technischen Nutzen der Synthesesequenz ist die hier bei den Steroiden vom Progesterontyp bestehende Möglichkeit, im Zuge des Reduktionsschritts von (5) nach (8) das 3-Keto-4-en-System des A-Rings in Form des Enamins (7) (oder auch als Ethylenketal) zu schützen und damit eine Überreduktion zu vermeiden, die leicht auch zur teilweisen Reduktion der Doppelbindung führen kann.

Die Übertragung einer solchen partiellen Blockierung des A-Rings auf das entsprechende Dehydroprogesteron und vergleichbare Dehydroprogesteronderivate ist technisch nicht möglich. Dehydroprogesteron und seine entsprechenden Derivate zeichnen sich durch die 3-Keto-1,4-dien-Struktur des A-Rings aus, wie in den folgenden Formeln (1a) bis (3a) angegeben :

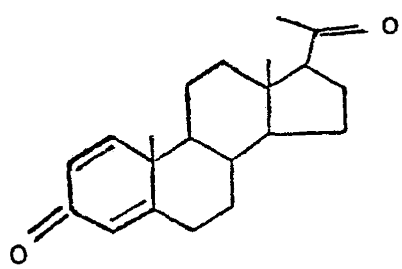

(1a)

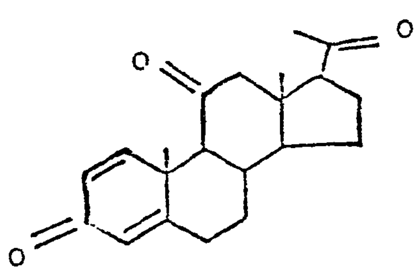

(2a)

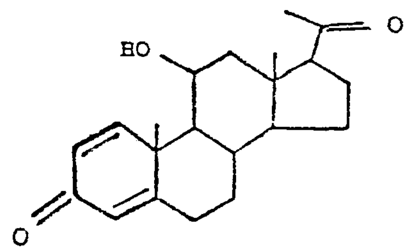

(3a)

Pregna-1,4-dien-3,20-dion (1a = Dehydroprogesteron), Pregna-1,4-dien-3,11,20-trion (2a) und Pregna-1,4-dien-11-ol-3,20-dion (3a), das sowohl als 11β- als auch als 11α-Isomeres vorliegen kann, besitzen eine beträchtliche potentielle Bedeutung als Vorstufen für die Herstellung von Prednisolon, Prednison und anderen Corticoiden mit 1,4-Dien-3-keto-struktur. Dehydroprogesteron und verschiedenartigste Derivate hiervon einschließlich der aufgezeigten Verbindungen (2a) und (3a) sind heute u. a. durch Abbau von Pregna-1,4-dien-3-on-20-carbonsäure (Δ1,4-BNC) und/oder ihrer funktionellen Derivate zugänglich. Diese Δ1,4-BNC und/oder ihre funktionellen Derivate sind auf mikrobiologischem Wege durch partiellen Abbau natürlicher tierischer oder pflanzlicher Steroidverbindungen wie Cholesterin oder Sitosterin zugänglich. Hierzu besteht eine Mehrzahl älterer Vorschläge der Anmelderin, siehe z. B. Europäische Patentanmeldung 004913 und 0015308.

Schwierigkeiten bereitet insbesondere in der Reihe der Steroidverbindungen mit 1,4-Dien-3-keto-Struktur die partielle Reduktion der C 21-Carbonsäuregruppierung zur entsprechenden C 21-Alkoholfunktion. Anlaß hierfür ist die mangelnde Schutzfähigkeit des A-Rings in Verbindungen der Dehydroprogesteron-Reihe.

Zwar beschreiben J. Hogg et al. (J. Am. Soc. 77, 4438 (1955)), daß die zuvor in den Formelbildern (5) bis (8) an Progesteronderivaten dargestellte Reaktionsfolge auf den entsprechenden Dehydroprogesteronester mit zusätzlicher 1(2)-Doppelbindung gegenüber (5) angewandt werden könne. Die erst viel später (Steroids 3, 189 (1964)) publizierte Ausbeute beträgt aber nur ca. 12 % (maximal 20 %) gegenüber einer Ausbeute von mehr als 70 % für die Umwandlung von (5) zu (8).

In jüngerer Zeit wird schließlich angegeben — siehe hierzu J. Fried, J.A. Edwards « Organic Reactions in Steroid Chemistry », Band 1, Seiten 394/395 (Van Nostrand Reinhold Company, New York, 1972), daß es für $\Delta^{1,4}$-3-Keto-Systeme in Steroiden keine brauchbare Schutzgruppe gibt.

Will man also ausgehend von den Dehydroprogesteronen, beispielsweise der Formeln (1a) bis (3a) Prednisolon-Vorstufen in technisch brauchbaren Ausbeuten herstellen, so ist die für die Hydrocortisonsynthese angegebene Reaktionsfolge nicht geeignet. Durch die Zugänglichkeit der Verbindungen (1a) bis (3a) und weiterer Dehydroprogesteronderivate aus der Δ1,4-BNC und ihren Verbindungen und die große Bedeutung, die Prednisolon, Prednison und andere Corticoide mit 1,4-Dien-3-keto-Struktur besitzen, ist ein solches Herstellungsverfahren jedoch wünschenswert.

Die vorliegende Erfindung geht von der Aufgabe aus, einen Weg aufzuzeigen, auf dem eine partielle Reduktion einer C-21-Carbonsäurefunktion in der C 17-Seitenkette von Steroidverbindungen möglich wird, auch wenn an sich vergleichsweise empfindliche weitere funktionelle Gruppen im Steroidringgerüst vorliegen. Als solche weiteren funktionellen Gruppen sind insbesondere die $\Delta^{4}$-3-on-Struktur, die $\Delta^{1,4}$-3-on-Struktur, weitere Doppelbindungen im System, insbesondere in 17(20)-Stellung und gegebenenfalls 9(11)-Stellung sowie mögliche Sauerstoff-Funktionen in 11-Stellung zu nennen. Besondere Bedeutung kommt bei all diesen Verbindungen Ausgangsmaterialien zu, die im A-Ring des Steroidgerüsts die $\Delta^{1,4}$-3-on-Struktur besitzen. Die Erfindung hat sich weiterhin die Aufgabe gestellt, eine partielle oder wenigstens weitgehend partielle Reduktion der Carboxylgruppen-funktion in C 21 ohne Mitverwendung besonderer getrennter Schutzgruppen für die A-Ring-Struktur zu ermöglichen.

Gegenstand der Erfindung ist dementsprechend ein Verfahren zur partiellen Reduktion von Δ4,17(20) — sowie gegebenenfalls weitere Doppelbindungen in 1(2)- und/oder 9(11)-Stellung aufweisenden C 21-Steroidcarbonsäuren und ihren Estern der allgemeinen Formel I

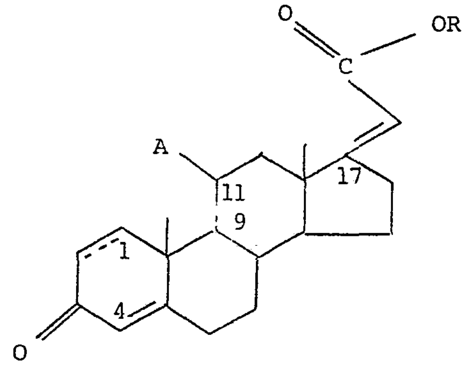

(I)

in der R Wasserstoff oder einen Kohlenwasserstoffrest und A Wasserstoff, Hydroxyl oder zusammen mit dem durch A substituierten C-Atom eine Carbonylgruppe bedeuten und wobei schließlich auch anstelle des Substituenten A eine zusätzliche olefinische Doppelbindung in 9(11)-Stellung vorliegen kann, zu C 21-Steroidalkoholen der allgemeinen Formel II

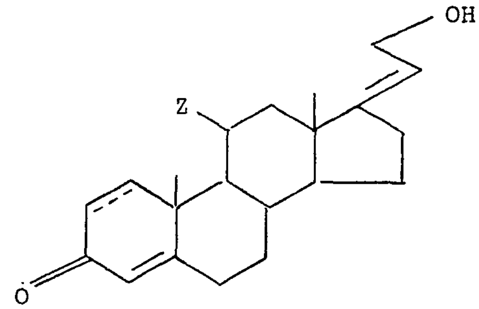

(II)

in der Z die Bedeutung von A hat, hierbei jedoch anstelle der Carbonylgruppe Hydroxyl bedeutet. Das erfindungsgemäße Verfahren ist dadurch gekennzeichnet, daß man die im A-Ring des Steroidsystems ungeschützte Ausgangsverbindung der allgemeinen Formel I mit Diisobutyl-Aluminiumhydrid (DIBAH) in solchen Mengen umsetzt, daß die Reduktion aller O-haltigen funktionellen Gruppen zur Hydroxylgruppe stattfindet, woraufhin das Aluminium-haltige Reaktionszwischenprodukt in an sich bekannter Weise der selektiven Oppenauer-Oxidation zur 3-Keto-Verbindung unterworfen wird.

Die erfindungsgemäß gesuchte selektive Reduktionsmöglichkeit in hohen Ausbeuten an der Carboxylgruppenfunktion in C 21 gelingt bei Verbindungen der allgemeinen Formel I überraschend einfach mit Diisobutyl-Aluminiumhydrid (DIBAH) als Reduktionsmittel. Am Beispiel der Reduktion des Esters (9) zum Alkohol (10) soll dies näher erläutert werden. Der Alkohol (10) selbst ist eine neue bisher nicht bekannte Verbindung und fällt in den Rahmen der Erfindung.

Die Reduktion wird ohne eine Schutzgruppe für das 1,4-Dien-3-keto-System des A-Rings durchgeführt. Stattdessen wird das Reduktionsmittel in einer Menge angewendet, die sowohl die Estergruppe als auch die 3-Keto-Gruppe reduziert. Dabei werden weder die Doppelbindungen des A-Rings, noch die 17(20)-Doppelbindung angegriffen.

Das Alkoxyaluminiumderivat (11), welches als Zwischenstufe angenommen werden kann, wird üblicherweise nicht isoliert, sondern unter geeigneten milden Bedingungen direkt einer selektiven Oppenauer-Oxidation durch Zugabe eines Alkohol/Keton-Gemisches (z. B. Isopropanol/Aceton) unterworfen.

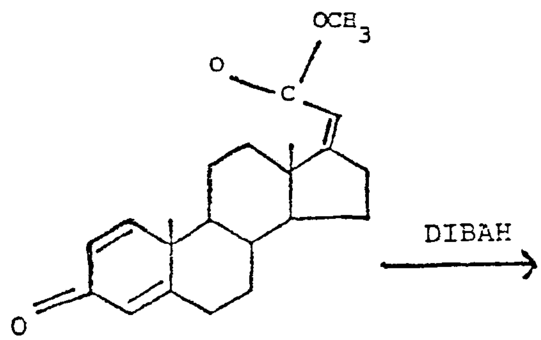


(9)

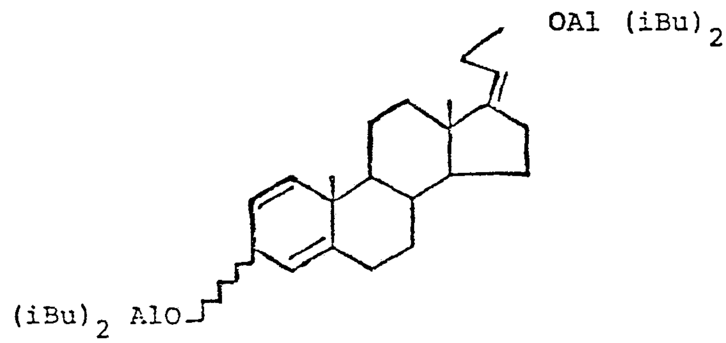

(11)

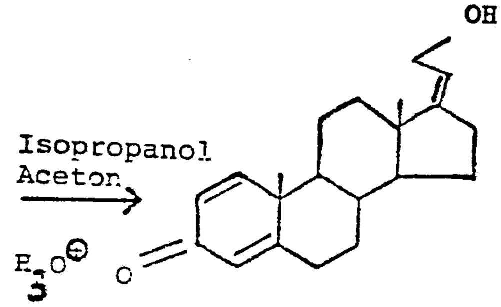


(10)

Dabei wird praktisch ausschließlich die 3-Keto-Gruppe zurückgebildet und man erhält den C 21-Alkohol (10) in isolierter Ausbeute von mehr als 70 %.

R In den Verbindungen der allgemeinen Formel I ist bevorzugt ein Kohlenwasserstoffrest mit insbesondere nicht mehr als 20 C-Atomen, zweckmäßig nicht mehr als 10 C-Atomen. Aliphatische Reste, insbesondere solche mit 1-5, vorzugsweise 1-3 C-Atomen sind besonders bevorzugt. Der wichtigste Rest ist der Methylrest.

Die erfindungsgemäße partielle Reduktion der Carboxyl- bzw. Carboxylatgruppe in C 21 ist auch bei Verbindungen möglich, die der allgemeinen Formel I entsprechen und in 9(11)-Stellung eine zusätzliche olefinische Doppelbindung enthalten. Das gleiche ist der Fall bei Verbindungen der allgemeinen Formel I, in denen A eine Hydroxylgruppe in α- oder insbesondere in β-Stellung bedeutet. Liegt bei den erfindungsgemäß eingesetzten Ausgangsverbindungen der allgemeinen Formel I in A eine Carbonylgruppe vor — d. h. bildet A mit dem durch A substituierten C-Atom in 11-Stellung eine Carbonylgruppe — dann wird allerdings auch diese Carbonylgruppe zur entsprechenden Hydroxylgruppe reduziert. Es fällt die 11β-Hydroxylgruppe an, die ohnehin aus pharmakologischen Gründen besondere Bedeutung besitzt. Ansonsten gilt aber auch hier, wie bei allen anderen Ausgangsverbindungen der allgemeinen Formel I daß die Struktur des A-Ringes nach Abschluß der 2-stufigen Reduktion und selektiven Oxidation unverändert aus dem Verfahren hervorgeht, wobei dieses besondere technische Bedeutung für Verbindungen mit der $\Delta^{1,4}$-3-on-Struktur im A-Ring hat.

Die Anwendung von Diisobutylaluminiumhydrid als Reduktionsmittel in der Steroidchemie ist an sich nicht neu. Vorbeschrieben ist sie für einige ausgewählte Steroidverbindungen mit der 4-En-3-keto-Struktur. So schildert U. Eder Chem. Ber. 109, 2954 (1976) ein Verfahren zur Herstellung von Testosteron aus 4-Androsten-3,17-dion durch Reduktion mit DIBAH und anschließende selektive Oppenauer-Oxidation. Auf diese Weise gelingt die selektive Reduktion der Keto-Gruppierung in 17-Stellung. Auch die Umwandlung des Δ4-BNC-Methylesters in den entsprechenden Δ4-BNC-Alkohol auf diesem Wege wird geschildert. Diese bekannten Vorschläge setzen jedoch Verbindungen ein, die eine einfachere Struktur als die erfindungsgemäß verwendeten Verbindungen besitzen. So weisen die erfindungsgemäß eingesetzten Ausgangsmaterialien die eine Doppelbindung in der Seitenkette auf, besitzen darüberhinaus in der bevorzugten Ausführungsform die Δ1,4-Dien-Struktur anstelle des einfacheren Δ4-Systems und können außerdem eine Sauerstoff-Funktion in 11-Stellung oder eine weitere Doppelbindung in 9(11)-Stellung besitzen. Es war daher nicht mit Sicherheit vorherzusehen, daß die von Eder beschriebene Reaktion auf die erfindungsgemäß eingesetzten Verbindungen übertragbar sein würde.

In C.A. 63, 13367 (1965) wird die Verbesserung der eingangs geschilderten Reduktion der Verbindungen der Formel (5) zum Diol der Formel (8) durch Verwendung von DIBAH anstelle von $LiAlH_4$ geschildert. Hier handelt es sich jedoch um Reduktion an einem durch Enamin-Bildung geschützten 4-En-3-keto-System — vergl. die zuvor dargestellten Formeln (6) und (8).

Im folgenden werden Einzelheiten zur Durchführung des erfindungsgemäßen Verfahrens gegeben :

Die Darstellung der ungesättigten Ausgangsmaterialien der allgemeinen Formel I, die im erfindungsgemäßen Verfahren als Ausgangsmaterial dienen, erfolgt in guten Ausbeuten aus den entsprechenden Verbindungen vom Progesteron- bzw. Dehydroprogesterontyp analog zum vorhergezeigten allgemeinen Formelschema der Verbindung der Formel (4) zur Verbindung der Formel (5). Einzelheiten zu dieser Reaktion sind der bereits zitierten Literatur, insbesondere J. A. Hogg et al., J. Am. Chem. Soc. 77, 4436 (1955) zu entnehmen.

Die Reduktion mit Diisobutylaluminiumhydrid erfolgt im Rahmen des an sich bekannten Wissens, vergl. hierzu beispielsweise E. Winterfeldt « Synthesis » 1975, Seite 617 ff.

Die Reaktion erfolgt üblicherweise im Temperaturbereich von etwa − 80 °C bis + 30 °C, wobei der Bereich von − 30 °C bis Raumtemperatur bevorzugt ist. Besonders geeignet ist der Temperaturbereich von − 10 bis + 5 °C. Die Umsetzung erfolgt vorzugsweise in Gegenwart inerter Lösungsmittel, beispielsweise aromatischer Kohlenwasserstoffe, wie Toluol oder Benzol, geeignet sind aber ebenfalls aliphatische Kohlenwasserstoffe, wie Hexan, oder Ether, wie Diethylether und dergleichen. Die Reaktionszeit beträgt üblicherweise bis 10 Stunden und liegt vorzugsweise im Bereich von 2 bis 5 Stunden. Zweckmäßigerweise wird die Reaktion unter Schutzgas, beispielsweise unter $N_2$, durchgeführt. Es wird in wasserfreier Atmosphäre gearbeitet. Es kann bevorzugt sein, eine Lösung von DIBAH im inerten Lösungsmittel zur Lösung der Steroidverbindung in einem weiteren Anteil des inerten Lösungsmittels zu

geben, aber auch die umgekehrte Arbeitsweise ist möglich. Die Menge des eingesetzten DIBAH entspricht wenigstens der stöchiometrisch benötigten Menge, vorzugsweise wird mit einem leichten Überschuß, beispielsweise bis zu 20 %, vorzugsweise bis zu ca. 10 % molarem Überschuß gearbeitet. Zur Reduktion der Carboxylgruppenfunktion in C 21 sind 2 Äquivalentanteile DIBAH erforderlich, jede Ketogruppe benötigt einen Äquivalentanteil DIBAH.

Zur anschließenden selektiven Oppenauer-Oxidation ist die Isolierung des intermediär gewonnenen aluminiumhaltigen Reaktionsproduktes nicht erforderlich. In der Regel ist es stattdessen bevorzugt, das primär erhaltene Reduktionsprodukt unmittelbar weiter in der anschließenden Oppenauer-Oxidation aufzuarbeiten. Hierfür gelten wiederum die generellen Angaben der einschlägigen Literatur, siehe hierzu beispielsweise Carl Djerassi in Organic Reactions, Band VI, Kapitel 5 « The Oppenauer-Oxidation » (J. Wiley and Sons, Inc., New York, 1951). Im einzelnen gilt hier insbesondere :

Die Reaktionstemperatur liegt üblicherweise im Bereich von etwa 0 bis 40 °C, kann aber auch darüber hinaus gehen. Zweckmäßigerweise wird unterhalb von 80 °C gearbeitet. Als Oxidationsmittel werden Keton/Alkohol/Gemische eingesetzt. Geeignete Hydridakzeptoren sind beispielsweise Aceton, Cyclohexanon oder Fluorenon in Abmischung mit Isopropanol oder tert. Butanol als Alkoholkomponente. Die Reaktionszeit beträgt hier üblicherweise 2 bis 20 Stunden, vorzugsweise 4 bis 12 Stunden. Die Reaktion wird ebenfalls unter wasserfreien Bedingungen durchgeführt.

Üblicherweise wird das Keton im großen Überschuß eingesetzt, der beispielsweise das 5- bis 50-fache, vorzugsweise das 10- bis 20-fache der stöchiometrisch benötigten Menge beträgt, um das Gleichgewicht der Reaktion in die erwünschte Richtung zu verschieben.

Durch das erfindungsgemäße Verfahren sind sowohl neue wie an sich bekannte Verbindungen herstellbar. Neu sind im Rahmen der erfindungsgemäß erhaltenen ungesättigten Alkohole gemäß der allgemeinen Formel II die Verbindungen, die im A-Ring des Steroidgerüstes die $\Delta^{1,4}$-3-on Konfiguration aufweisen und in 11-Stellung unsubstituiert sind oder auch in 9(11)-Stellung eine weitere Doppelbindung besitzen. Diese beiden neuen C 21-ol-Verbindungen sind als solche ein weiterer Gegenstand der Erfindung.

Die erfindungsgemäß erhaltenen Verbindungen der allgemeinen Formel II sind wertvolle Produkte der Steroidchemie und insbesondere wertvolle Zwischenprodukte bei der Herstellung pharmakologisch aktiver Steroidverbindungen, insbesondere der Prednison- und/oder Prednisolonreihe. Auch die Anwendung der erfindungsgemäß erhaltenen Verbindungen der allgemeinen Formel II für diesen zuletzt genannten Zweck fällt in den Rahmen der vorliegenden Erfindung und ist damit ein weiterer Gegenstand der hier beanspruchten technischen Lehre.

Für diese Umwandlung der C 21-ol-Verbindungen zu Produkten, z. B. der Prednison- bzw. Prednisolonreihe, wird in der Regel zunächst die Hydroxylgruppe in C 21 verestert, insbesondere acetyliert. Die Acetylierung gelingt beispielsweise durch Umsetzung der C 21-ol-Verbindung mit Acetanhydrid/Pyridin oder Acetanhydrid/Triäthylamin/Dimethylaminopyridin. In dieser Stufe des Verfahrens fallen wiederum teils neue, teils an sich bekannte Verbindungen an. Neue Verbindungen — und damit ein weiterer Gegenstand der vorliegenden Erfindung — sind das Acetat des Pregna-1,4,17(20)-trien-3-on-21-ol sowie der entsprechenden Verbindung mit einer zusätzlichen olefinischen Doppelbindung in 9(11)-Stellung. Bekannte Acetate dieser Art sind entsprechende Verbindungen mit der $\Delta^4$-3-on-Konfiguration und/oder einer Sauerstoff-Funktion in 11-Stellung.

Die Umwandlung dieser in der C 21-Hydroxylgruppe veresterten Verbindungen zu Verbindungen z. B. des Typs von Prednison bzw. Prednisolon erfolgt in an sich bekannter Weise. Verwiesen wird hier auf die bereits zitierte Literaturstelle J. A. Hogg et al., J. Am. Chem. 77, Seiten 4436 ff., insbesondere S. 4438. Im einzelnen erfolgt hier eine oxidative Hydroxylierung mit Osmiumtetroxid, wobei zusätzliche Sauerstoff-Funktionen in C 17 und C 20 eingeführt werden. Bei der oxidativen Hydroxylierung in tert.-Butylalkohol/Pyridin mit Phenyliodosoacetat in Gegenwart katalytischer Mengen von Osmiumtetroxid entsteht aus 11β-Hydroxy-4,17(20)-pregna-dien-3-on-21-acetat Hydrocortisonacetat, das in an sich bekannter Weise in Hydrocortison umgewandelt werden kann. Bei Ausgangsverbindungen mit der Δ1,4-Dien-3-on-Struktur fallen letztlich Verbindungen der Prednison- bzw. Prednisolonreihe an.

## Beispiel 1

Pregna-1,4,17(20)-trien-21-ol-3-on.

Zu 3 g des Esters Pregna-1,4,17(20)-cis-trien-3-on-21-säuremethylester [1)] in 60 ml abs. Toluol werden bei 0 °C 25,4 ml einer 20 %igen Lösung von Diisobutylaluminiumhydrid in Toluol getropft. Nach 3 h bei 0 °C fügt man 9 ml Aceton, dann 9 ml Isopropanol zu und läßt die Lösung auf Raumtemperatur kommen. Nach Stehen über Nacht säuert man bei 0 °C vorsichtig mit 10 %iger Schwefelsäure an. Nach der Phasentrennung wird die wäßrige Phase 2 x mit wenig Methylenchlorid extrahiert, die organischen Phasen werden getrennt, neutral gewaschen, danach gemeinsam über $Na_2SO_4$ getrocknet und nach Abtrennen des Trockenmittels zur Trockne eingeengt. Der Rückstand wird über Kieselgel mit Methylenchlorid/Essigester (75 : 25) chromatographiert. Man erhält 2,04 g (72 %) des Produkts, die nach Dünnschichtchromatogramm und $^1$H-NMR-Spektrum einheitlich sind.

Fp (Ether) : 105-108 °C

$C_{21}H_{28}O_2$ gef. : C 80,46 %, H 9,15 %
ber. : C 80,73 %, H 9,03 %

$^1$H-NMR (80 MHz, $CDCl_3$, chemische Verschiebung ist angegeben in ppm in der δ-Skala, bezogen auf TMS) :

0,96 (18-$CH_3$, s) ; 1,23 (19-$CH_3$, s) ; 4,23 (21-$CH_2$, m) ; 5,32 (20-CH, m) ;

6,07
6,15 6,17
6,27 6,30
6,99 7,11
} ABC-System von 1-CH, 2-CH und 4-CH

Aus der 17(20)-cis-Konfiguration des Esters[1)] wird diese auch für den Alkohol abgeleitet.

1) B.J. Magerlein und J. A. Hogg, J. Amer, Chem. Soc. 80, 2220 (1958)

## Beispiel 2

21-Acetoxy-pregna-1,4,17(20)-trien-3-on

400 mg des nach Beispiel 1 hergestellten Pregna-1,4,17(20)-trien-21-ol-3-on in 10 ml abs. Methylenchlorid werden mit 0,24 ml Acetanhydrid, 0,36 ml Triethylamin und 50 mg 4-Dimethylaminopyridin versetzt und bei 0 °C 30 min gerührt. Wie ein Dünnschichtchromatogramm zeigt, ist die Umsetzung nach dieser Zeit quantitativ. Man wäscht die Reaktionslösung nacheinander mit eiskalter verdünnter HCl, $NaHCO_3$-Lösung und schließlich mit Wasser, trocknet die $CH_2Cl_2$-Phase über $Na_2SO_4$, filtriert vom Trockenmittel ab und engt zur Trockne ein. Der Rückstand ist praktisch reines Produkt in über 90 %iger Ausbeute.

$^1$H-NMR (80 MHz, $COCl_3$ ; δ-Werte) :

0,96 (18-$CH_3$, S) ; 1,21 (19-$CH_3$, S) ; 2,01 ($-\overset{O}{\overset{\|}{C}}-CH_3$, S) ; 4,63 (21-$CH_2$, d J = 7,2 Hz) ; 5,24 (20-CH, m) ;

6,04
6,12 6,14
6,23 6,25
6,98 7,08
} ABC-System von 1-CH, 2-CH und 4-CH

$C_{23}H_{30}O_3$ gef : C 77,69 %, H 8,36 %
ber : C 77,93 %, H 8,53 %

## Beispiel 3

11β-21-Dihydroxy-pregna-1,4,17(20)-trien-3-on

3 g Pregna-1,4,17(20)-trien-3,11-dion-21-säuremethylester werden wie in Beispiel 1 beschrieben mit 32,4 ml 20 %iger DIBAH-Lösung reduziert. Nach 3 h bei 0 °C gibt man bei dieser Temperatur 11,1 ml Aceton, danach 11,1 ml Isopropanol zu, läßt über Nacht bei Raumtemperatur stehen, erwärmt dann noch 1 h auf 40 °C und arbeitet auf und reinigt wie in Beispiel 1 beschrieben.

Man erhält 1,67 g (59 %) des Alkohols vom Fp 174-178 °C (Lit.[1] : 174-175 °C).

Setzt man den Ester mit $\Delta^4$-A-Ring ein, so gelingt die Reduktion zum Alkohol mit $\Delta^4$-A-Ring unter den gleichen Bedingungen und praktisch gleicher Ausbeute wie in Beispiel 3.

Der Schmelzpunkt (Fp) des Reaktionsproduktes stimmte mit dem in der Literatur[2] angegebenen gut überein.

1. J. A. Hogg et al., J. Amer. Chem. Soc. 77, 4438 (1955)
2. J. A. Hogg et al., J. Amer. Chem. Soc. 77, 4436 (1955)

## Ansprüche

1. Verfahren zur partiellen Reduktion von Δ4,17(20) — sowie gegebenenfalls weitere Doppelbindungen in 1- und/oder 9(11)-Stellung aufweisenden C 21-Steroidcarbonsäuren und ihren Estern der allgemeinen Formel I

(Siehe Formel I, Seite 9 f.)

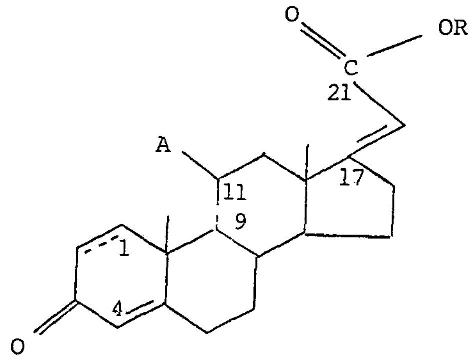

(I)

in der R Wasserstoff oder einen Kohlenwasserstoffrest und A Wasserstoff, Hydroxyl, oder zusammen mit dem durch A substituierten C-Atom eine Carbonylgruppe bedeuten und wobei schließlich auch anstelle des Substituenten A eine zusätzliche olefinische Doppelbindung in 9(11)-Stellung vorliegen kann, zu C 21-Steroidalalkoholen der allgemeinen Formel II

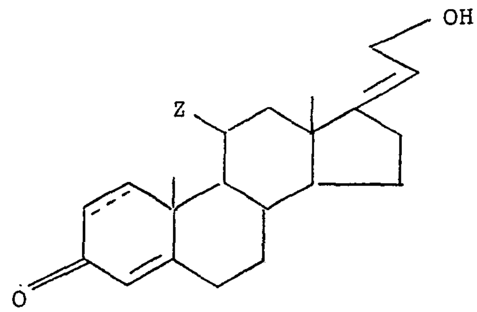

(II)

in der Z die Bedeutung von A hat, hierbei jedoch anstelle einer Carbonylgruppe Hydroxyl bedeutet, dadurch gekennzeichnet, daß man die im A-Ring des Steroidringsystems ungeschützte Ausgangsverbindung der allgemeinen Formel I unter wasserfreien Bedingungen im Temperaturbereich von − 80 °C bis + 30 °C mit Diisobutylaluminium-hydrid in solchen Mengen umsetzt, daß die Reduktion aller sauerstoffhaltigen funktionellen Gruppen zur Hydroxylgruppe stattfindet, woraufhin das aluminiumhaltige Reaktionszwischenprodukt in an sich bekannter Weise der selektiven Oppenauer-Oxidation durch Umsetzung des primär erhaltenen Reduktionsproduktes mit einem Keton-Alkoholgemisch unter wasserfreien Bedingungen bei Temperaturen unterhalb von 80 °C zur 3-Keto-Verbindung unterworfen wird.

2. Verfahren nach Anspruch 1, dadurch gekennzeichnet, daß die Umsetzung mit Diisobutylaluminiumhydrid unter wasserfreien Bedingungen im Temperaturbereich von − 10 °C bis + 5 °C, zweckmäßigerweise in inerten Lösungsmitteln durchgeführt wird.

3. Verfahren nach Ansprüchen 1 und 2, dadurch gekennzeichnet, daß die selektive Oppenauer-Oxidation durch Umsetzung des primär erhaltenen Reduktionsproduktes mit einem Keton/Alkoholgemisch ebenfalls unter wasserfreien Bedingungen bei Temperaturen zwischen 0 und 40 °C erfolgt, wobei bevorzugt das Keton in großem Überschuß über die stöchiometrisch benötigte Menge eingesetzt wird.

**Claims**

1. A process for the partial reduction of Δ4,17(20)-C 21-steroid carboxylic acids optionally containing other double bonds in the 1- and/or 9(11)-position and their esters corresponding to the following general formula

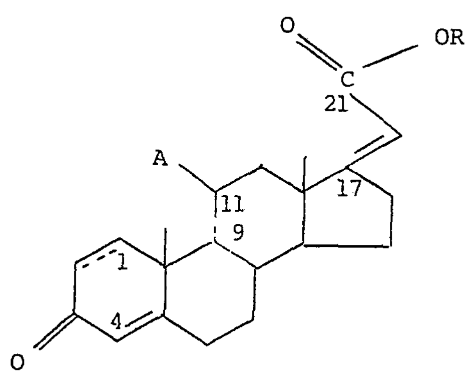

(I)

in which R represents hydrogen or a hydrocarbon radical and A represents hydrogen, hydroxyl or, together with the C-atom substituted by A, a carbonyl group and in which, finally, an additional olefinic double bond may be present in the 9(11)-position in place of the substituent A, to form $C_{21}$-steroid alcohols corresponding to the following general formula

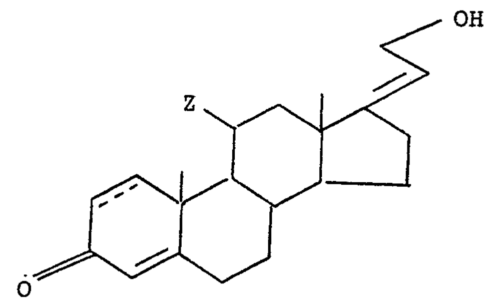

(II)

in which Z has the same meaning as A, but instead of a carbonyl group represents a hydroxyl group, characterized in that the starting compound of general formula I, which is unprotected in the A-ring of the steroid ring system, is reacted with diisobutyl aluminium hydride under anhydrous conditions at temperatures in the range from − 80 °C to + 30 °C in such quantities that all the oxygen-containing functional groups are reduced to the hydroxyl group, after which the aluminium-containing intermediate reaction product is subjected in known manner to selective Oppenauer oxidation by reacting the reduction product primarily obtained with a ketone-alcohol mixture under anhydrous conditions at temperatures below 80 °C to form the 3-keto compound.

2. A process as claimed in Claim 1, characterized in that the reaction with diisobutyl aluminium hydride is carried out under anhydrous conditions at temperatures in the range from − 10 °C to + 5 °C, advantageously in inert solvents.

3. A process as claimed in Claims 1 and 2, characterized in that the selective Oppenauer oxidation is carried out by reacting the reduction product obtained with a ketone/alcohol mixture, again under anhydrous conditions, at temperatures in the range from 0 to 40 °C, the ketone preferably being used in a large excess over and above the stoichiometrically necessary quantity.

**Revendications**

1. Procédé pour la réduction partielle d'acides stéroïde-21-carboxyliques et de leurs esters contenant des doubles liaisons Δ4,17(20) et éventuellement d'autres doubles liaisons dans les positions 1- et/ou 9(11) et répondant à la formule générale I

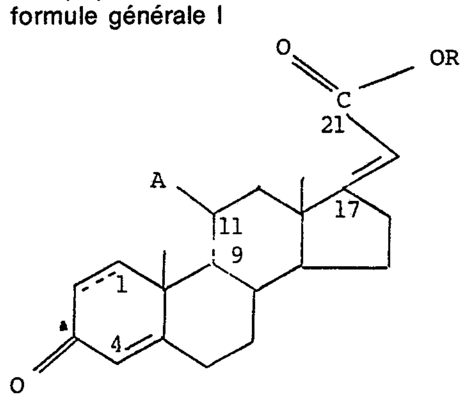

(I)

dans laquelle R représente l'hydrogène ou un reste hydrocarboné et A représente l'hydrogène, un groupe hydroxy ou forme avec l'atome de carbone substitué par A un groupe carbonyle et dans lesquels finalement, à la place du substituant A, il peut exister une double liaison oléfinique supplémentaire en position 9(11), en stéroïde-21-alcools de formule générale II

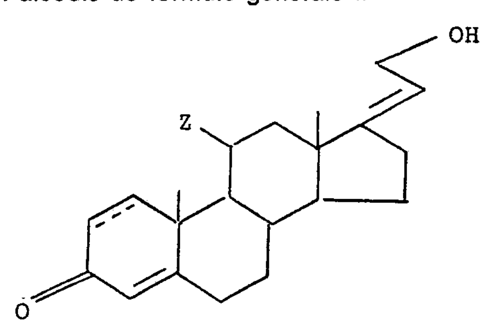

(II)

dans laquelle Z a la signification de A, mais représente toutefois, à la place d'un groupe carbonyle, un groupe hydroxy, caractérisé en ce que l'on fait réagir le composé de départ de formule générale I non protégé dans le cycle A du système cyclique stéroïdique, en milieu anhydre, dans l'intervalle de température de − 80 à + 30 °C, avec l'hydrure de diisobutyl-aluminium en quantité telle qu'il y ait réduction de tous les groupes fonctionnels contenant de l'oxygène en groupes hydroxy, après quoi on soumet le produit intermédiaire de réaction contenant de l'aluminium à l'oxydation sélective d'Oppenauer de manière connue en soi, par réaction du produit de réduction obtenu en premier avec un mélange cétone-alcool en milieu anhydre à des températures inférieures à 80 °C, ce qui donne le composé 3-cétonique.

2. Procédé selon la revendication 1, caractérisé en ce que la réaction avec l'hydrure de diisobutyl-aluminium est effectuée en milieu anhydre dans un intervalle de température de − 10 à + 5 °C, de préférence dans des solvants inertes.

3. Procédé selon les revendications 1 et 2, caractérisé en ce que l'oxydation sélective d'Oppenauer par réaction du produit de réduction obtenu en premier avec un mélange cétone/alcool est également effectuée en milieu anhydre à des températures de 0 à 40 °C, avec de préférence utilisation de la cétone en gros excès par rapport à la quantité théorique nécessaire.